# EUROPEAN PATENT SPECIFICATION

(11) **EP 3 261 672 B1**
(45) Date of publication and mention of the grant of the patent: **05.07.2023**
(21) Application number: 16756416.0
(22) Date of filing: 26.02.2016
(51) Int. Cl.: C07K 16/40, C07K 14/515, A61K 39/395, A61P 11/00, A61P 1/16

(54) **MOLECULES DISRUPTING PYRUVATE KINASE M2 AND INTEGRIN INTERACTION AND USES THEREOF**
MOLEKÜLE ZUR UNTERBRECHUNG DER PYRUVATKINASE M2 UND INTEGRININTERAKTION UND VERWENDUNGEN DAVON
MOLÉCULES PERTURBANT LA PYRUVATE KINASE M2 ET L'INTERACTION DE L'INTÉGRINE ET LEURS UTILISATIONS

(30) Priority: 26.02.2015 US 201562121338 P
(43) Date of publication of application: 03.01.2018
(73) Proprietor: Proda Biotech LLC, Marietta, GA 30068 (US)
(72) Inventor: LIU, Zhi-ren, Marietta, Georgia 30068 (US); LI, Liangeiwei, Atlanta, Georgia 30329 (US); ZHANG, Yinwei, Decatur, Georgia 30033 (US)
(74) Representative: Dr. Träger & Strautmann PAe PartG mbB
(86) International application number: PCT/US2016/019705
(87) International publication number: WO 2016/138346

(56) References cited:
- WO-A1-90/05917
- WO-A1-2013/123193
- US-A- 5 972 628
- US-A1- 2007 117 966
- US-A1- 2010 099 726
- Anonymous: "Product datasheet Anti-PKM2 antibody [EPR10138(B)] ab150377; Rabbit monoclonal" In: "Product datasheet Anti-PKM2 antibody [EPR10138(B)] ab150377; Rabbit monoclonal", 15 November 2013 (2013-11-15), XP055486016, * the whole document * -& XU BAOZENG ET AL: "Bi-directional communication with the cumulus cells is involved in the deficiency of XY oocytes in the components essential for proper second meiotic spindle assembly", DEVELOPMENTAL BIOLOGY, ELSEVIER, AMSTERDAM, NL, vol. 385, no. 2, 15 November 2013 (2013-11-15), pages 242-252, XP028548980, ISSN: 0012-1606, DOI: 10.1016/J.YDBIO.2013.11.004
- Li Lingwei: "Extracellular Pyruvate Kinase M2 regulates tumor angiogenesis" In: "Extracellular Pyruvate Kinase M2 regulates tumor angiogenesis", 10 May 2014 (2014-05-10), United States, XP055485905, pages 1-209, * p 141-147; p 36, Fig 2A-G, Fig 3D-F; Fig. S5B; Fig. 7; p 101, bottom *
- VIBHOR GUPTA ET AL: "Human pyruvate kinase M2: A multifunctional protein : Multifunctional Human PKM2", PROTEIN SCIENCE, vol. 19, no. 11, 26 October 2010 (2010-10-26), pages 2031-2044, XP055485975, US ISSN: 0961-8368, DOI: 10.1002/pro.505
- Yinwei Zhang: "FUNCTIONS OF EXTRACELLULAR PYRUVATE KINASE M2 IN TISSUE REPAIR AND REGENERATION", Dissertation at Georgia State University, 1 May 2016 (2016-05-01), page e104157, XP055486165, United States DOI: 10.1371/journal.pone.0104157 Retrieved from the Internet: URL:https://scholarworks.gsu.edu/cgi/viewc ontent.cgi?referer=https://www.google.com/ &httpsredir=1&article=1172&context=biology _diss [retrieved on 2018-06-20]
- MICHAEL S. GOLDBERG ET AL: "Pyruvate kinase M2-specific siRNA induces apoptosis and tumor regression", THE JOURNAL OF EXPERIMENTAL MEDICINE, vol. 209, no. 2, 23 January 2012 (2012-01-23), pages 217-224, XP055486408, US ISSN: 0022-1007, DOI: 10.1084/jem.20111487
- Yinwei A Zhang: "Functions of Extracellular Pyruvate Kinase M2 in Tissue Repair and Regeneration" In: "Dissertation, Georgia State University, 2016.", 9 May 2016 (2016-05-09), XP055485994, pages 1-171,
- KOSUGI, M ET AL.: 'MUC1-C Regulates Glycolysis and PKM2.' PLOS ONE vol. 6, no. 11., pages 1 - 14, XP009505608
- Zhaofei Liu ET AL: "Integrin [alpha] v &bgr; 3 -targeted cancer therapy", Drug development research, vol. 69, no. 6, 1 September 2008 (2008-09-01), pages 329-339, XP055069888, ISSN: 0272-4391, DOI: 10.1002/ddr.20265
- Lara H. El Touny ET AL: "The Role of Fibrosis in Tumor Progression and the Dormant to Proliferative Switch" In: "Tumor Dormancy, Quiescence, and Senescence, Volume 2", 5 November 2013 (2013-11-05), Springer Netherlands, Dordrecht, XP055611648, ISBN: 978-94-007-7726-2 pages 155-164, DOI: 10.1007/978-94-007-7726-2_16,
- CHANDLER CHELSEA ET AL: "The double edge sword of fibrosis in cancer", TRANSLATIONAL RESEARCH, vol. 209, 21 February 2019 (2019-02-21), pages 55-67, XP055907405, NL ISSN: 1931-5244, DOI: 10.1016/j.trsl.2019.02.006
- Patsenker Eleonora ET AL: "Pharmacological inhibition of integrin [alpha]v[beta]3 aggravates experimental liver fibrosis and suppresses hepatic angiogenesis", Hepatology, vol. 50, no. 5, 2 July 2009 (2009-07-02), pages 1501-1511, XP055907586, US ISSN: 0270-9139, DOI: 10.1002/hep.23144
- GIAN LUCA BAGNATO ET AL: "The double edge sword of fibrosis in cancer", CLINICAL SCIENCE., vol. 132, no. 2, 13 December 2017 (2017-12-13), pages 231-242, XP055597990, GB ISSN: 0143-5221, DOI: 10.1042/CS20171426
- MATTEVI A ET AL: "Crystal structure of Escherichia coli pyruvate kinase type I: molecular basis of the allosteric transition", STRUCTURE, ELSEVIER, AMSTERDAM, NL, vol. 3, no. 7, 1 July 1995 (1995-07-01), pages 729-741, XP004587882, ISSN: 0969-2126, DOI: 10.1016/S0969-2126(01)00207-6

## Description

### TECHNICAL FIELD

The presently disclosed subject matter generally relates to composition for use in a method for treating diseases associated with aberrant integrin αvβ3 expression in a subject. The method relates to administering to the subject an effective amount of a composition comprising a pyruvate kinase isoform M2 (PKM2) antagonist that inhibits the binding of PKM2 to integrin αvβ3.

It is explicitly noted, that any reference in the description to methods of treatment refer to the compounds, pharmaceutical compositions and medicaments of the present invention for use in a method for treatment of the human (or animal) body by therapy.

WO 2013/123193 A1 is directed to methods for inhibiting angiogenesis such as tumor angiogenesis in a subject based on PKM2 inhibition. WO 90/05917 A1 discloses an anti-pyruvate kinase M2 mAb used in the treatment of tumors. Lingwei, Li, "Extracellular Pyruvate Kinase M2 Regulates Tumor Angiogenesis", 10 May 2014, page 1-209, discloses that PKM2 enhances angiogenesis by promoting endothelial cell migration, adhesion and survival via interaction with integrin a5b3. Patsenker et al., "Pharmacological Inhibition of Integrin avb3 Aggravates Experimental Liver Fibrosis and Suppresses Hepatic Angiogenesis", Hepatology, vol. 50, no. 5, 2 July 2009, pages 1501-1511, DOI: 10.1002/hep.23144, discusses specific inhibition of integrin avb3 in vivo decreased angiogenesis as well as adverse effects.

### BACKGROUND

A substantial and persuasive body of data now exists that supports the view that integrin αᵥβ₃ plays a critical part in activated macrophage dependent inflammation, osteoclast development, migration, and bone resorption, and inflammatory angiogenesis. All of these processes play an important part in the pathogenesis of rheumatoid arthritis (RA) and related arthropathies. Therefore, agents are needed that specifically inhibit aberrant integrin αᵥβ₃ activity.

### SUMMARY

The invention is set out in the claims. As disclosed herein, pyruvate kinase isoform M2 (PKM2) promotes myofibroblast differentiation by acting on integrin αᵥβ₃. A composition for use in a method is disclosed for disrupting or preventing the interaction of PKM2 and integrin αᵥβ₃. The method involves administering to a subject an effective amount of a composition comprising a PKM2 antagonist that inhibits the binding/interaction of PKM2 to integrin αᵥβ₃. The disclosed composition can be used to treat diseases that involve aberrant integrin αᵥβ₃ expression, wherein the claimed composition for use in a method treats a fibrotic disease, such as interstitial liver fibrosis or pulmonary fibrosis as disclosed in claim 1.

### DESCRIPTION OF DRAWINGS

The following examples do not specifically define the scope of the invention which is done by the claims but are suited to illustrate specific aspects helpful for understanding the invention.

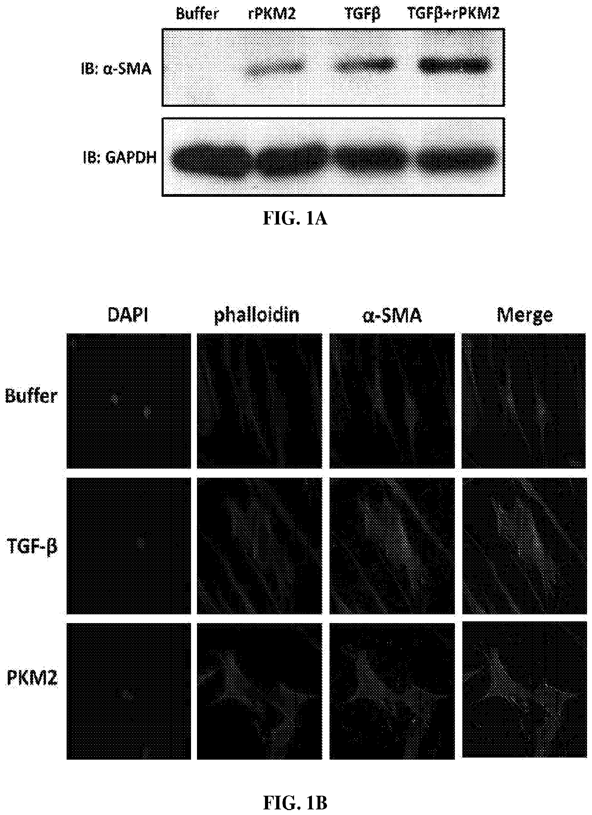

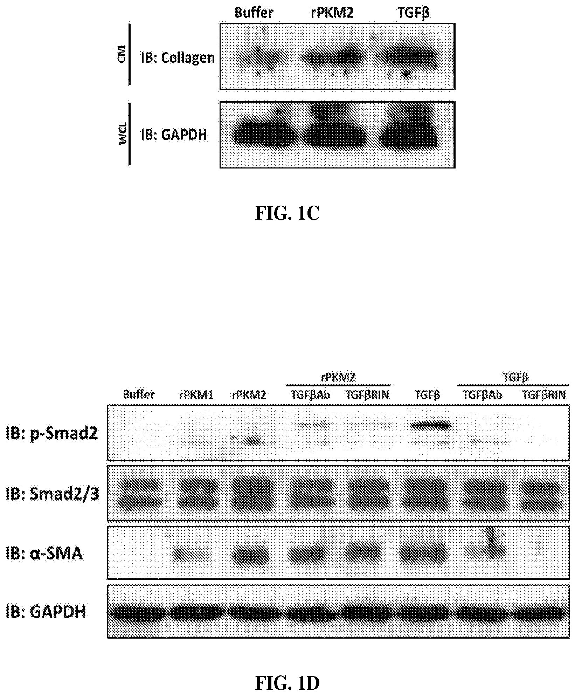

**FIG. 1** shows images of PKM2 promoting myofibroblast differentiation, including FIG. 1A in which cellular levels of α-SMA in HDFa cells were analyzed by immunoblot of α-SMA (IB:a-SMA), FIG. 1B of representative images of IF staining of HDFa cells with anti-α-SMA (green), rhodamine Phalloidin (red), and DAPI (blue), FIG. 1C in which cells were treated with the indicated agents showing on the left, FIG. 1D in which the cellular levels of phosphorylated Smad2, Smad2/3, and α-SMA in HDFa cells that were treated by the indicated agents were analyzed by the immunoblots (IB:p-Smad2, IB:Smad2/3, and IB:α-SMA).

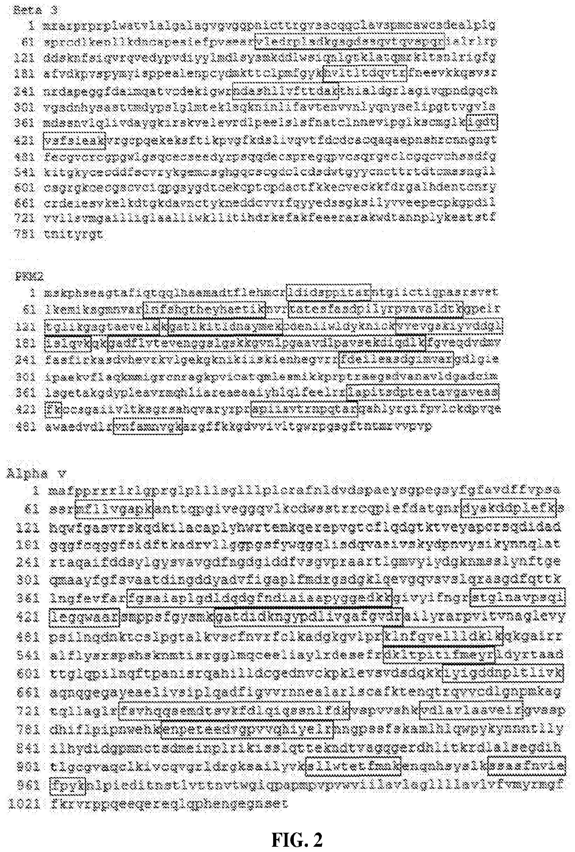

**FIG. 2** includes graph and images showing extracellular PKM2 activates integrin αᵥβ₃ signaling. The peptide fragments (underlined) from trypsin digestion of the His-tag pulled-down crosslinking band of rPKM2-HDFa crosslinks and analyzed by MALDI-tof/tof that match the amino acid sequence of integrin β₃ and PKM2.

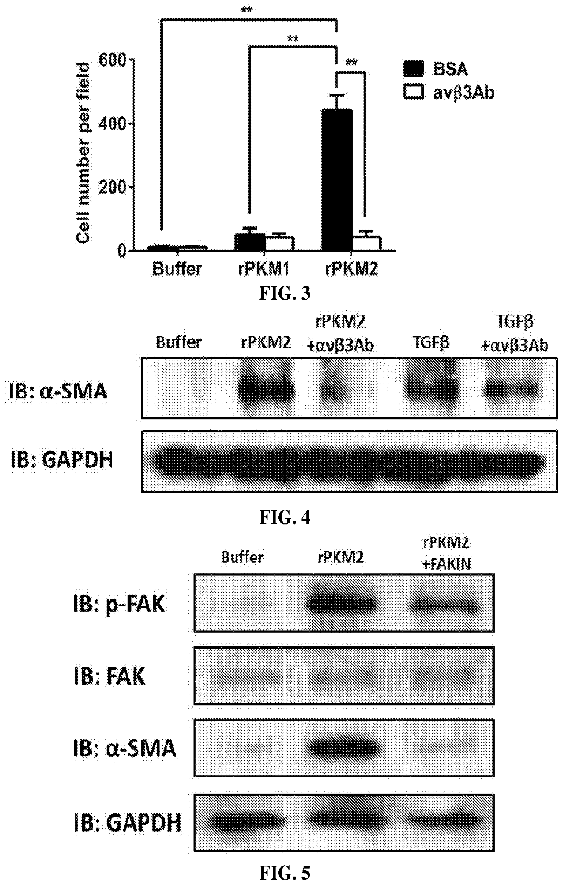

**FIG. 3** includes bar graphs showing the attachment of HDFa cells to buffer, rPKM1, and rPKM2 coated plate in the presence of 5 µg/ml of LM609 (open bars) or BSA (filled bars). The attachment is presented as total number of cells attached to the plate per view field. Error bars are standard deviations from measurement of five independent experiments.
**FIG. 4** includes images showing the cellular levels of α-SMA in HDFa cells were analyzed by immunoblot of α-SMA (IB:α-SMA). The cells were treated by the indicated agents.
**FIG. 5** includes images showing Cellular levels of phosphorylated FAK in HDFa cells were examined using antibody against the phosphorylated FAK (IB:p-FAK). The cells were treated by indicated agents. Immunoblot of FAK (IB:FAK) indicates the levels of total FAK. In (C) and (D), Immunoblot of GAPDH is a loading control.

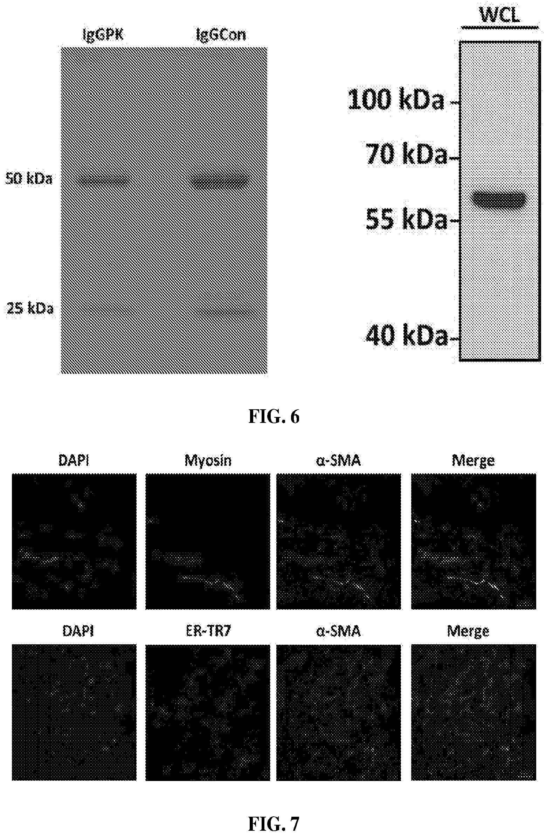

**FIG. 6** includes images illustrating the effects of PKM2 antibody on angiogenesis at wound site. (Left) IgG purified from monoclonal antibody IgGPK or rabbit pre-immuno serum (IgGCon) by protein G/A beads. (Right) Specific recognition of PKM2 by the purified IgG of IgGPK in SW620 cell extracts.
**FIG. 7** includes images showing the α-SMA staining co-localize with fibroblast ER-TR7 staining. Representative images of immunostaining of wound tissue sections with antibodies against mouse α-SMA (green), mouse smooth muscle myosin heavy chain (Upper, red), and ER-TR7 (Bottom, red). Blue are DAPI staining. Merge is overlay of three color staining. Tissue sections were prepared from tissues samples of wound three days post-wound induction.

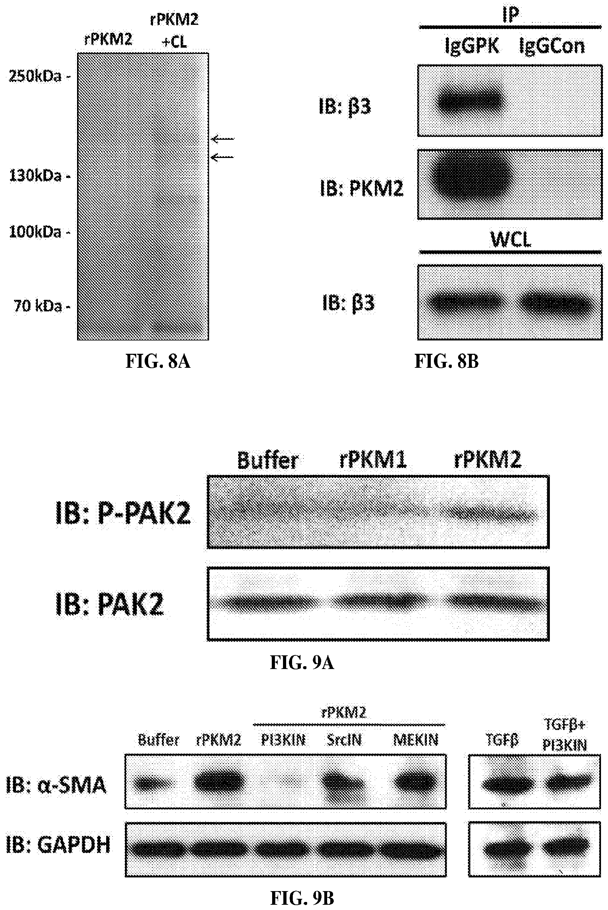

**FIG. 8** includes images showing that PKM2 acts via interaction with integrin αᵥβ₃. FIG. 8A shows crosslinking of rPKM2 with HDFa cell surface proteins was revealed by immunoblot of PKM2 (IB:PKM2). The rPKM2 was crosslinked to HDFa cells by glutaraldhyde. The extracts were prepared from the crosslinked cells. His-tagged rPKM2 was pulled down from the prepared extracts by Ni-beads. Ni beads precipitations were analyzed by immunoblot. FIG. 8B is co-immunoprecipitation of integrin β₃ with PKM2 (IP:IgGPK) was examined by immunoblot of integrin β₃ (IB: β₃). Immunoblot of PKM2 (IB:PKM2) indicates amounts of PKM2 are precipitated down. Immunoprecipitation using IgG purified from pre-immune serum (IP:IgGCon) is control immunoprecipitation. Immunoblot of IgG heave chain (IgG HC) indicated amount of IgG used in each immunoprecipitation.
**FIG. 9** include images illustrating that PKM2 acts via activation of PI3K. FIG. 9A illustrates activation of p21-dependent kinase 2 (PAK2) in HDFa cells in the presence of indicated agents was analyzed immunoblot of phosphorylated PAK2 (IB:P-PAK2). Immunoblot of total cellular PAK2 (IB:PAK2) is a control indicating total cellular levels of PAK2. FIG. 9B shows expression of α-SMA in HDFa cells under the indicated treatments were analyzed by immunoblot of α-SMA (IB: D-SMA). The immunoblot of GAPDH (IB:GPDH) is a loading control.

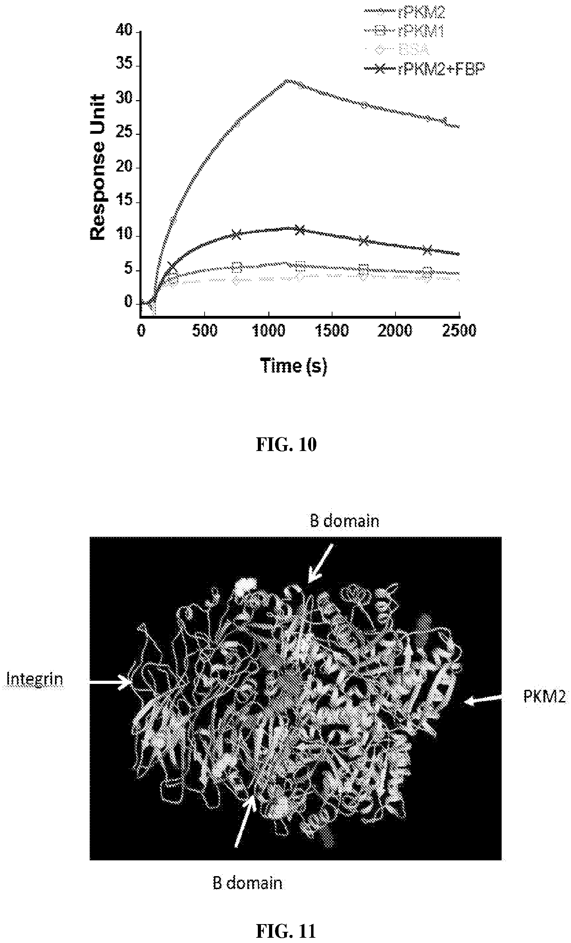

**FIG. 10** is a graph showing the representative binding curves of binding of rPKM2, rPKM1, BSA, and rPKM2 in the presence of FBP to integrins αᵥβ₃ that were monitored by Biacore with integrins that were immobilized on the Biacore chip.
**FIG. 11** is a diagram illustrating docking of PKM2 dimer on integrin αᵥβ₃ head-piece. The docking model was computed based on the chemical crosslinks between rPKM2 and recombinant integrin αᵥβ₃.

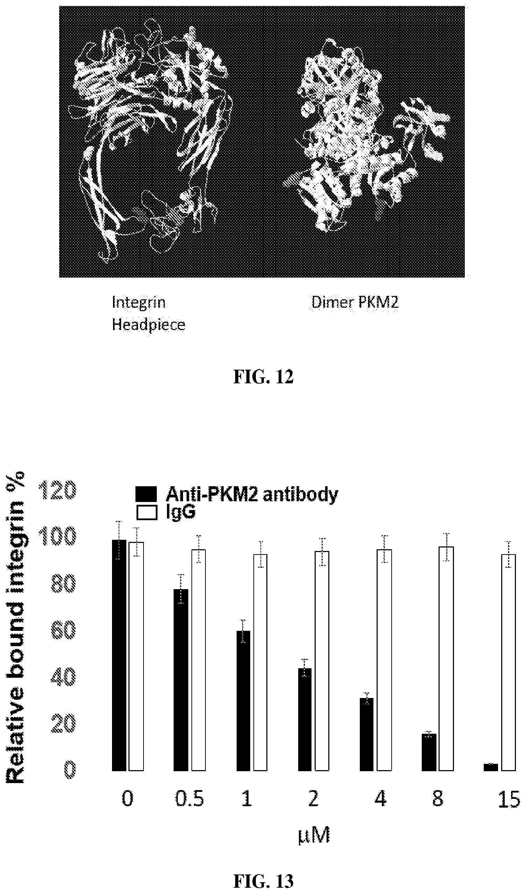

**FIG. 12** includes diagrams illustrating chemical crosslinking map between rPKM2 dimer and integrin αᵥβ₃ headpiece. The same colored residues represent each corresponding crosslink pair between the integrin and PKM2.
**FIG. 13** includes bar graphs showing ELISA analyses of binding of integrin αᵥβ₃ to rPKM2 in the presence of a monoclonal antibody against PKM2 (Filled bars) and rabbit IgG (Open bars). The rPKM2 3 µM was first coated on the plate. Various concentration of antibody or IgG was mixed with integrin αᵥβ₃ were added to the plate. After extensive washing, binding of integrin αᵥβ₃ to the coated rPKM2 was determined by second antibody against the integrin as ELISA read-out, and presented as relative bound integrin by defining the bound integrin in the absence of the antibody or IgG as 100%. The error bars represent standard deviations from five repeating experiments.

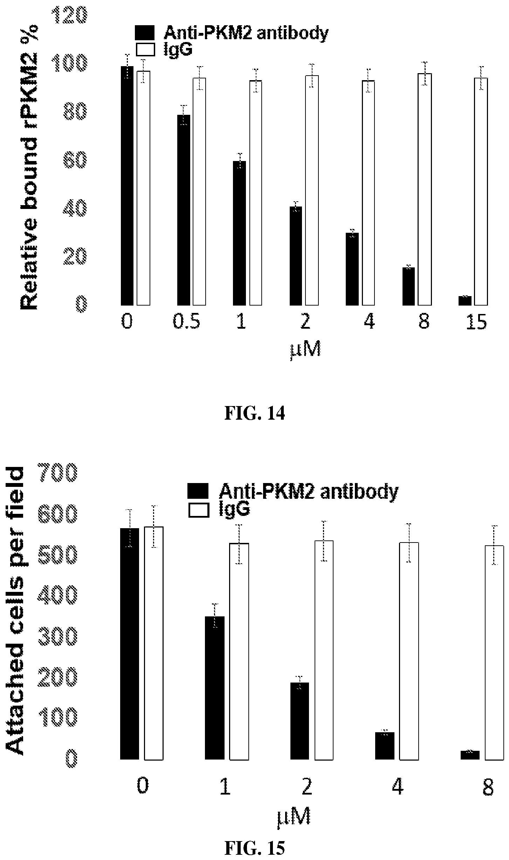

**FIG. 14** includes bar graphs showing ELISA analyses of binding of integrin αᵥβ₃ to rPKM2 in the presence of a monoclonal antibody against PKM2 (Filled bars) and rabbit IgG (Open bars). The recombinant integrin αᵥβ₃ 3 µM was first coated on the plate. Various concentration of antibody or IgG was mixed with rPKM2 were added to the plate. After extensive washing, binding of rPKM2 to the coated integrin αᵥβ₃ was determined by second antibody against PKM2 as ELISA read-out, and presented as relative bound rPKM2 by defining the bound rPKM2 in the absence of the antibody or IgG as 100%. The error bars represent standard deviations from five repeating experiments.
**FIG. 15** includes bar graphs showing the attachment of HUVEC cells to PKM2 (3 µM) coated plate in the presence of the anti-PKM2 antibody (Filled bars) or rabbit IgG (Open bars) was analyzed by cell counting after extensive washing. The attached cells were counted per microscopic field (average of four fields). The error bars represent standard deviations from five repeating experiments.

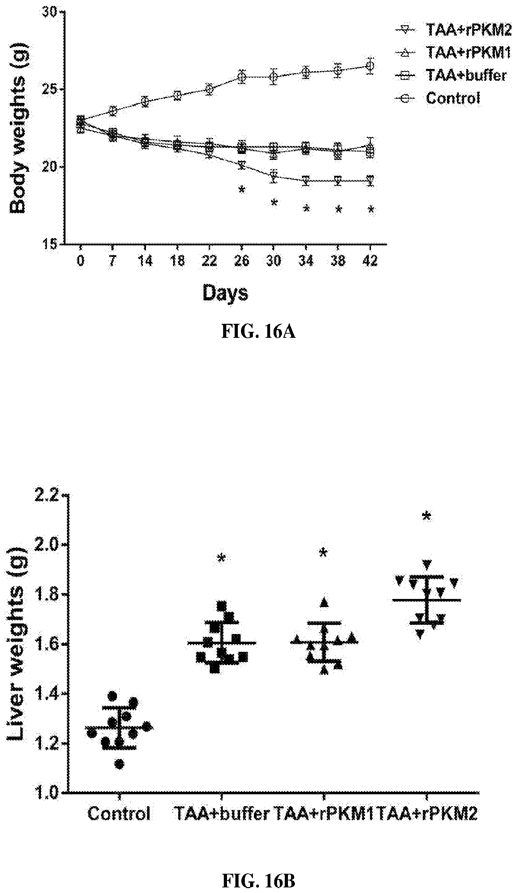

**FIG. 16** includes graphs showing that PKM2 facilitates liver fibrosis progression.

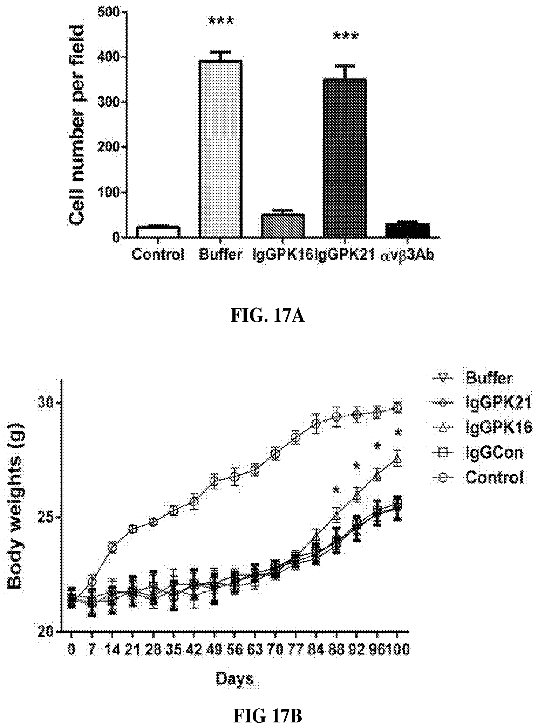

**FIG. 17** includes graphs showing that a treatment consisting of PKM2 antibody reversed liver fibrosis.

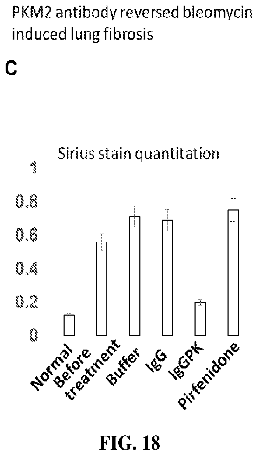

**FIG. 18** PKM2 antibody reversed bleomycin induced lung fibrosis

Lung fibrosis is induced by bleomycin.

### DEFINITIONS

The term "fibrosis" refers to any disease or condition involving excess fibrous connective tissue in an organ or tissue.

The term "neutralize" refers to the ability of an agent, such as an antibody, to specifically bind a ligand and in so doing block or inhibit the ligand's biological activity. A neutralizing antibody is an antibody that inhibits or abolishes some biological activity of its target antigen.

The term "antibody" refers to natural or synthetic antibodies that binds or selectively bind a target antigen. The term includes polyclonal and monoclonal antibodies. In addition to intact immunoglobulin molecules, also included in the term "antibodies" are fragments or polymers of those immunoglobulin molecules, and human or humanized versions of immunoglobulin molecules that selectively bind the target antigen.

A "monoclonal antibody" can be obtained from a substantially homogeneous population of antibodies, i.e., the individual antibodies within the population are identical except for possible naturally occurring mutations that may be present in a small subset of the antibody molecules. Monoclonal antibodies include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, as long as they exhibit the desired antagonistic activity. Monoclonal antibody can be derived from species including but not limited to rabbits and mice.

The terms "peptide," "protein," and "polypeptide" are used interchangeably to refer to a natural or synthetic molecule comprising two or more amino acids linked by the carboxyl group of one amino acid to the alpha amino group of another.

"Activities" of a protein include, for example, transcription, translation, intracellular translocation, secretion, phosphorylation by kinases, cleavage by proteases, homophilic and heterophilic binding to other proteins, ubiquitination.

The term "peptidomimetic" refers to a mimetic of a peptide which includes some alteration of the normal peptide chemistry. Peptidomimetics typically enhance some property of the original peptide, such as increase stability, increased efficacy, enhanced delivery, increased half life, etc. Methods of making peptidomimetics based upon a known polypeptide sequence is described, for example, in U.S. Patent Nos. 5,631,280; 5,612,895; and 5,579,250. Use of peptidomimetics can involve the incorporation of a non-amino acid residue with non-amide linkages at a given position.

The term "specifically binds" refers to a binding reaction which is determinative of the presence of the antigen or receptor in a heterogeneous population of proteins and other biologies. Generally, a first molecule (e.g., antibody) that "specifically binds" a second molecule (e.g., antigen) has an affinity constant (Ka) greater than about 10⁵ M⁻¹ (e.g., 10 M⁻¹, 10⁷ M⁻¹, 10⁸ M⁻¹, 10⁹ M⁻¹, 10¹⁰ M⁻¹, 10¹¹ M⁻¹, and 10¹² M⁻¹ or more) with that second molecule.

The term "subject" refers to any individual who is the target of administration or treatment. The subject can be a vertebrate, for example, a mammal. Thus, the subject can be a human or veterinary patient. The term "patient" refers to a subject under the treatment of a clinician, e.g., physician.

The term "therapeutically effective" refers an amount of composition that is sufficient to ameliorate one or more causes or symptoms of a disease or disorder. Such amelioration only requires a reduction or alteration, not necessarily elimination of the disease or disorder.

The term "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problems or complications commensurate with a reasonable benefit/risk ratio.

The term "treatment" refers to the medical management of a patient with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder.

The term "inhibit" refers to a decrease in an activity, response, condition, disease, or other biological parameter. This can include but is not limited to the complete ablation of the activity, response, condition, or disease. This may also include, for example, a 10% reduction in the activity, response, condition, or disease as compared to the native or control level. or control levels.

### DETAILED DESCRIPTION

A composition comprising a pyruvate kinase isoform M2 (PKM2) antagonist for use in a method of treating a disease is disclosed according to claim 1.

With respect to FIGs. 1-18, specific embodiments include PKM2 antagonists for use in the disclosed compositions and methods as claimed. Certain embodiments include PKM2 antagonists that specifically bind PKM2 at domain A (α-helix 6' to α -helix 6, and α-helix 7'), domain B: (β -sheet 1 and β -sheet 5), and/or domain C (α-helix 5 to β-sheet 4) (collectively and individually referred to as "PKM2 Binding Sites" and which inhibits the ability of PKM2 to bind integrin αᵥβ₃ in the subject. These structural features or domains are well characterized and can be found e.g., in Mattevi, A. et al., Structure 3, 729-741 (1995).

### Antibodies/PKM2 Antagonist

The PKM2 or PKM2 Binding Sites antagonist is an antibody. Antibodies that can be used in the disclosed compositions and methods include whole immunoglobulin (i.e., an intact antibody) of any class, fragments thereof, and synthetic proteins containing at least the antigen binding variable domain of an antibody. The variable domains differ in sequence among antibodies and are used in the binding and specificity of each particular antibody for its particular antigen. However, the variability may not be evenly distributed through the variable domains of antibodies and may be concentrated in three segments called complementarity determining regions (CDRs) or hypervariable regions both in the light chain and the heavy chain variable domains. The more highly conserved portions of the variable domains are called the framework (FR). The variable domains of native heavy and light chains each comprise four FR regions, largely adopting a beta-sheet configuration, connected by three CDRs, which form loops connecting, and in some cases forming part of, the beta- sheet structure. The CDRs in each chain are held together in close proximity by the FR regions and, with the CDRs from the other chain, contribute to the formation of the antigen binding site of antibodies. Therefore, the disclosed antibodies contain at least the CDRs necessary to bind PKM2 and inhibit the binding/interaction of PKM2 and integrin αᵥβ₃.

Methods for generating monoclonal antibodies, including fully human monoclonal antibodies are known in the art. Any such known methods can be used in the context of this application to make human antibodies that specifically bind to PKM2 or PKM2 Binding Sites. Using known method for generating monoclonal antibodies, high affinity chimeric antibodies to PKM2 or PKM2 Binding Sites are initially isolated having a human variable region and a mouse constant region. As in the experimental section below, the antibodies are characterized and selected for desirable characteristics, including affinity, selectivity, epitope, etc. The mouse constant regions are replaced with a desired human constant region to generate the fully human antibody of the invention, for example wild-type or modified IgG1 or IgG4. While the constant region selected may vary according to specific use, high affinity antigen-binding and target specificity characteristics reside in the variable region.

To screen for antibodies which bind to a particular epitope (e.g., those which block binding of IgE to its high affinity receptor), a routine cross-blocking assay such as that described in Harlow and Lane (1990) supra can be performed. Other methods include alanine scanning mutants, peptide blots (Reineke (2004) Methods Mol Biol 248:443-63), or peptide cleavage analysis. In addition, methods such as epitope excision, epitope extraction and chemical modification of antigens can be employed (Tomer (2000) Protein Science 9:487-496).

Any further aspects, examples and embodiments discussed herein, which are not specifically claimed, are included for illustration purposes only.

In a specific embodiment, the PKM2 antagonist is an antibody including a heavy chain variable region and a light chain variable region. The antibody specifically binds to PKM2 at domain A (α-helix 6' to α -helix 6, and α-helix 7'), the antibody binds to domain B (β -sheet 1 and β -sheet 5), the antibody binds to domain C (α-helix 5 to β-sheet 4). In some embodiments, the antibody specifically recognizes example sequence elements and other sequences of PKM2 that are involved in interacting with integrin aᵥb₃. Non-limiting examples of the specific sequences include Domain A a-helix 6, ARGDLGIEIPAEKVFLAQKMMIGRCNR and a-helix 7' QMLESMIKKP, Domain B beta-sheet 1 EVELKKGAT and beta-sheet 5 ISLQVKQKGA, and Domain C a-helix 5 DVDLRVNFAMNVGKA and beta-sheet 4 KKGDVVIVLTGWR.

In one specific embodiment, the PKM2 antagonist is capable of binding the following sequences of PKM2: Domain A helix 6 (ARGDLGIEIPAEKVFLAQKMMIGRCNR) and α-helix 7' (QMLESMIKKP), Domain B β-sheet 1 (EVELKKGAT) and β-sheet 5 (ISLQVKQKGA) Domain C α-helix 5 (DVDLRVNFAMNVGKA) and β-sheet 4 (KKGDVVIVLTGWR).

In some embodiments, the presently disclosed subject matter relates to an antibody comprising a heavy chain variable region and a light chain variable region, wherein said antibody specifically binds to at least one PKM2 domain selected from a group consisting of domain A, domain B and domain C.

Also disclosed are fragments of antibodies which have bioactivity. The fragments, whether attached to other sequences or not, include insertions, deletions, substitutions, or other selected modifications of particular regions or specific amino acids residues, provided the activity of the fragment is not significantly altered or impaired compared to the nonmodified antibody or antibody fragment.

Techniques can also be adapted for the production of single-chain antibodies specific for PKM2. A single chain antibody can be created by fusing together the variable domains of the heavy and light chains using a short peptide linker, thereby reconstituting an antigen binding site on a single molecule. Single-chain antibody variable fragments (scFvs) in which the C-terminus of one variable domain is tethered to the N-terminus of the other variable domain via a 15 to 25 amino acid peptide or linker have been developed without significantly disrupting antigen binding or specificity of the binding. The linker is chosen to permit the heavy chain and light chain to bind together in their proper conformational orientation.

Divalent single-chain variable fragments (di-scFvs) can be engineered by linking two scFvs. This can be done by producing a single peptide chain with two VH and two VL regions, yielding tandem scFvs. ScFvs can also be designed with linker peptides that are too short for the two variable regions to fold together (about five amino acids), forcing scFvs to dimerize. Diabodies have been shown to have dissociation constants up to 40-fold lower than corresponding scFvs, meaning that they have a much higher affinity to their target. Still shorter linkers (one or two amino acids) lead to the formation of trimers (triabodies or tribodies). Tetrabodies have also been produced. They exhibit an even higher affinity to their targets than diabodies.

Optionally, the antibodies are generated in other species and "humanized" for administration in humans. Antibodies can be humanized with retention of high affinity for the antigen and other favorable biological properties. To achieve this goal, humanized antibodies can be prepared by a process of analysis of the parental sequences and various conceptual humanized products using three dimensional models of the parental and humanized sequences. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the consensus and import sequence so that the desired antibody characteristic, such as increased affinity for the target antigen(s), is achieved. In general, the CDR residues are directly and most substantially involved in influencing antigen binding.

The targeting function of the antibody can be used therapeutically by coupling the antibody or a fragment thereof with a therapeutic agent. Such coupling of the antibody or fragment (e.g., at least a portion of an immunoglobulin constant region (Fc)) with the therapeutic agent can be achieved by making an immunoconjugate or by making a fusion protein, comprising the antibody or antibody fragment and the therapeutic agent.

### Peptides

In some aspects of the disclosure, but not claimed, the PKM2 antagonist is a peptide. Peptides that specifically bind PKM2, a PKM2 Binding Site or integrin αᵥβ₃ can be identified using routine methods, such as phage display and yeast two-hybrid assays.

The disclosed peptides generally contain at least one segment that selectively binds PKM2 . The disclosed binding peptides can have a variety of lengths and structures as described herein. Generally, the lengths can range from peptide to polypeptide length, and all such lengths are encompassed as described herein. Merely for the sake of convenience, the disclosed peptides and polypeptides generally are referred to herein as "polypeptides" but it is intended that use of this term encompasses such compositions that could be considered peptides, unless the context clearly indicates otherwise..

In some aspects of the disclosure, but not claimed, the binding peptide is a non-active fragment of integrin αᵥβ₃, e.g., containing the binding/interaction site(s) for PKM2 but not other domains necessary for activation of integrin αᵥβ₃.

### Pharmaceutical Formulations

A pharmaceutical composition containing therapeutically effective amounts of one or more of the disclosed PKM2 or PKM2 Binding Site antagonists in a pharmaceutically acceptable carrier is disclosed. Pharmaceutical carriers suitable for administration of the disclosed PKM2 or PKM2 Binding Site antagonists include any such carriers known to those skilled in the art to be suitable for the particular mode of administration. According to the invention, the pharmaceutical composition comprises a molecule that specifically binds pyruvate kinase isoform M2 (PKM2) and inhibits the binding of PKM2 to integrin αᵥβ₃, and a pharmaceutically acceptable carrier. The molecule agent can include a neutralizing antibody, a humanized antibody, and a monoclonal antibody. The monoclonal antibody can have a heavy chain variable region and a light chain variable region, and wherein said antibody specifically binds to at least one PKM2 domain selected from a group consisting of domain A, domain B and domain C.

In addition, the compounds may be formulated as the sole pharmaceutically active ingredient in the composition or may be combined with other active ingredients. For example, the compounds may be formulated or combined with known NSAIDs, antiinflammatory compounds, steroids, and/or antibiotics.

The PKM2 antagonists may be formulated into suitable pharmaceutical preparations such as solutions, suspensions, tablets, dispersible tablets, pills, capsules, powders, or sustained release formulations.

In one specific embodiment, the compositions are formulated for single dosage administration. To formulate a composition, the weight fraction of compound is dissolved, suspended, dispersed or otherwise mixed in a selected carrier at an effective concentration such that the treated condition is relieved or one or more symptoms are ameliorated.

The active compound is included in the pharmaceutically acceptable carrier in an amount sufficient to exert a therapeutically useful effect in the absence of undesirable side effects on the patient treated. The therapeutically effective concentration may be determined empirically by testing the compounds in in vitro, ex vivo and in vivo systems, and then extrapolated therefrom for dosages for humans.

The concentration of active compound in the pharmaceutical composition will depend on absorption, inactivation and excretion rates of the active compound, the physico chemical characteristics of the compound, the dosage schedule, and amount administered as well as other factors known to those of skill in the art.

The dosage and schedule for administration of a therapeutic antibody used in the disclosed methods can be determined by one of skill in the art. For example, the dosage of the antibody can range from about 0.1 mg/kg to about 50 mg/kg, typically from about 1 mg/kg to about 25 mg/kg. In particular embodiments, the 4 antibody dosage is 1 mg/kg, 3 mg/kg, 5 mg/kg, 10 mg/kg, 15 mg/kg, 20 mg/kg or 25 mg/kg. The dosage schedule for administration of the antibody can vary depending on the desired aggressiveness of the therapy, as determined by the practitioner.

An exemplary treatment regime entails administration once per every two weeks or once a month or once every 3 to 6 months. Antibody is usually administered on multiple occasions. Intervals between single dosages can be weekly, monthly or yearly. Alternatively, antibody can be administered as a sustained release formulation, in which case less frequent administration is required. Dosage and frequency vary depending on the half-life of the antibody in the patient. In general, human antibodies show the longest half-life, followed by humanized antibodies, chimeric antibodies, and nonhuman antibodies. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and until the patient shows partial or complete amelioration of symptoms of disease. Thereafter, the patent can be administered a prophylactic regime.

### Methods

Methods are disclosed for inhibiting or preventing diseases associated with abberant integrin αᵥβ₃ in a subject that involve administering to the subject a composition containing an effective amount of PKM2 antagonist in a pharmaceutically acceptable recipient. In specific embodiments, the PKM2 antagonist specifically binds PKM2 and inhibits the binding/interaction of PKM2 to integrin αᵥβ₃ in the subject. In some of these embodiments, the composition contains an effective amount of PKM2 antagonist to inhibit the binding/interaction of PKM2 to integrin αᵥβ₃ by at least 10% to 100%, including by about 10%, 20%, 30%, 40%, 50%, 60%, 70%, 80%, 90%, or 100%.

On specific embodiment includes methods for treating a disease associated with aberrant integrin αvβ3 expression in a subject, comprising administering to the subject an effective amount of a composition comprising a pyruvate kinase isoform M2 (PKM2) antagonist, wherein the PKM2 antagonist inhibits the binding of PKM2 to integrin αvβ3. The PKM2 antagonist can be an antibody that selectively binds PKM2. The PKM2 antagonist can be a monoclonal antibody. The monoclonal antibody comprises a heavy chain variable region and a light chain variable region, and wherein said antibody specifically binds to at least one PKM2 domain selected from a group consisting of domain A, domain B and domain C. In some embodiments, the PKM2 antagonist is a peptide or peptidomimetic that selectively binds PKM2.

### Administration

The disclosed pharmaceutical compositions containing PKM2 binding molecules may be administered in a number of ways to achieve therapeutically effective amounts of the PKM2 binding molecules in the circulation of the subject. For example, the disclosed compositions can be administered intravenously, intraperitoneally, intramuscularly, subcutaneously, intracavity, or transdermally. The compositions may be administered parenterally, ophthalmically, vaginally, rectally, intranasally, or by inhalant.

Parenteral administration of the composition, if used, is generally characterized by injection. Injectables can be prepared in conventional forms, either as liquid solutions or suspensions, solid forms suitable for solution of suspension in liquid prior to injection, or as emulsions. A revised approach for parenteral administration involves use of a slow release or sustained release system such that a constant dosage is maintained.

The disclosed compositions may be administered prophylactically to patients or subjects who are at risk for diseases associated with abberant integrin αᵥβ₃. Thus, the method can further comprise identifying a subject at risk for fibrosis prior to administration of the disclosed compositions.

The exact amount of the compositions required will vary from subject to subject, depending on the species, age, weight and general condition of the subject, the severity of the allergic disorder being treated, the particular composition used, its mode of administration and the like. Thus, it is not possible to specify an exact amount for every composition. However, an appropriate amount can be determined by one of ordinary skill in the art using only routine experimentation given the teachings herein. For example, effective dosages and schedules for administering the compositions may be determined empirically, and making such determinations is within the skill in the art. The dosage ranges for the administration of the compositions are those large enough to produce the desired effect in which the symptoms disorder are effected. The dosage should not be so large as to cause adverse side effects, such as unwanted cross-reactions, anaphylactic reactions, and the like. Generally, the dosage will vary with the age, condition, sex and extent of the disease in the patient, route of administration, or whether other drugs are included in the regimen, and can be determined by one of skill in the art. The dosage can be adjusted by the individual physician in the event of any counter indications. Dosage can vary, and can be administered in one or more dose administrations daily, for one or several days. Guidance can be found in the literature for appropriate dosages for given classes of pharmaceutical products. A typical daily dosage of the antibody used alone might range from about 1 mg/kg to up to 100 mg/kg of body weight or more per day, depending on the factors mentioned above.

The frequency of dose will depend on the half-life of the molecule and the duration of its effect. If the molecule has a short half-life (e.g. 2 to 10 hours) it may be necessary to give one or more doses per day. Alternatively, if the molecule has a long half life (e.g. 2 to 15 days) it may only be necessary to give a dosage once per day, once per week or even once every 1 or 2 months.

### EXAMPLES

The following examples and embodiments, which are not specifically claimed, are included for illustration purposes only.

### Example 1: Pyruvate Kinase M2 Released by Infiltration Neutrophils at Wound Site Facilitates Wound Healing.

### Extracellular PKM2 promotes myofibroblast differentiation

Experiments were conducted to determine whether rPKM2 could induce myofibroblast differentiation of cultured fibroblast cells. rPKM2 was added to the culture medium of HDFa cells. IF staining of the cells revealed that extracellular PKM2 promotes formation of stress-fiber bundle of_α-SMA with a typical pattern of myofibroblast (Fig. 1B). Furthermore, addition of rPKM2 into culture of HDFa increased the collagen I secretion (Fig. 1C). TGFE TGFβ is the major soluble factor that stimulates myofibroblast formation at wound site. Immediately following cutaneous wound, TGFE TGFβ is secreted by the accumulated platelets to facilitate converting fibroblast to myofibroblast (Gillitzer R, et al. J Leukoc Biol. 2001 69(4):513-21; Werner S, et al. Physiol Rev. 2003 83(3):83570). In order to determine whether rPKM2 would augment the effects of TGFE TGFβ to facilitate myofibroblast differentiation, rPKM2 was added to the medium of HDFa in addition to TGFETGFβ. Clearly, rPKM2 strengthened effects of TGFE in induction of myofibroblast formation (Fig. 1A). To exclude the possibility that the effects of rPKM2 on myofibroblast differentiation were due to presence of residues TGFE or activation of TGFE receptor, an antibody that neutralizes TGFE and an inhibitor that inhibits TGFE receptor activation were used. Both the antibody and the inhibitor had no effects on the function of rPKM2 in upregulation of Dα-SMA (Fig. 1D). Furthermore, it is known that TGFETGFβ/TGFE TGFβ receptor facilitate myofibroblast differentiation via activation of Smad pathway. Experiments were therefore conducted to determine whether addition of rPKM2 into culture of HDFa cells led to activation of Smad. Immunoblots demonstrated that Smad was not activated by rPKM2 (Fig. 1D). The results suggest that the effects of extracellular PKM2 on myofibroblast differentiation is independent of TGFE signaling.

### PKM2 promotes myofibroblast differentiation by acting on integrin αᵥβ₃

The extracts were prepared from the rPKM2 crosslinked cells. The crosslinked proteins were separated by His-tag pull-down. Immunoblot of PKM2 revealed a rPKM2 crosslinked protein. The rPKM2 crosslinked protein band was sliced out and digested by trypsin, and subsequently analyzed by MALDI-tof/tof. The MS-analyses showed that rPKM2 crosslinked to integrin β₃ (Fig. 2). Interaction of PKM2 with the integrin was verified by co-immunoprecipitation of rPKM2 with β3 in HDFa cell extracts (Fig. 2). It is known that integrin αᵥ normally paired with β₃. A cell attachment assay was used to verify the PKM2 and integrin αᵥβ₃ interaction. HDFa cells were attached to culture plate with rPKM2 coating. The attachment were completely blocked by LM609, an antibody recognizes integrin αᵥβ₃ and blocks integrin activation (Fig. 3). Furthermore, the antibody LM609 abolished the effects of rPKM2 on both HDFa cell migration (Fig. 8B) and α-SMA upregulation in the cells. As a control, the antibody had much less effects on the activity of TGFE in regulation of α-SMA expression (Fig. 4). The effects of LM609 on the activity of rPKM2 supported the notion that extracellular PKM2 acted via cell surface integrins αᵥβ₃. Integrin αᵥβ₃ signaling play important role in myofibroblast differentiation, and dermal fibroblast cells expression high levels of integrin αᵥβ₃ (Gailit J, et al. Exp Cell Res. 1997 232(1): 118-26; Hinz B. Periodontol 2000. 2013 63(1):14-28). It was reasoned that PKM2 might facilitate myofibroblast differentiation via activation of integrin αᵥβ₃ signaling. Indeed, addition of rPKM2 into culture of HDFa cells led to a strong FAK activation (Fig. 5). A FAK inhibitor abrogated the effects of PKM2 in upregulation of α-SMA (Fig. 5).

An open question was how the extracellular PKM2 that acts on integrins αᵥβ₃ upregulates α-SMA expression. The regulatory effects was not mediated via Smad pathway. TGFβ can promote myofibroblast differentiation by a Smad independent pathway via activation of PI3K (Wilkes MC, et al. Cancer Res. 2005 65(22):10431-40). On the other hand, activation of integrin signaling leads to activation of PI3K (Wegener KL, et al. Mol Membr Biol. 2008 25(5):376-87).

### Example 2: Antibodies against PKM2 and use of the antibodies

### Antibodies interrupt the interaction between PKM2 and integrin αᵥβ₃.

PKM2 interacts with integrin αᵥβ₃, and activates the integrin signaling (Figs. 11 and 12). A docking model was computed based on the chemical crosslinks between rPKM2 and recombinant integrin αᵥβ₃. As shown in Figures 13 and 14, PKM2 interacts with integrin αᵥβ₃ by its surfaces formed by (1) two B-domains and the A- and C- domains from the same monomer, near the FBP binding site, and (2) the dimer-dimer interface. In addition, Figure 15 shows electron microscopic images of integrin αᵥβ₃ with/without rPKM2 binding.

### Assays for analyses the effective of antibody that interruption of PKM2 and integrin αᵥβ₃ interaction.

*ELISA assay.* Recombinant PKM2 was coated on ELISA plates. Recombinant integrin αᵥβ₃ along with test antibody or IgG (as control) were added into the PKM2 coated plates. Plates were washed several times. Binding of the integrin αᵥβ₃ to PKM2 were measured using antibody against integrin αᵥβ₃ (Fig. 13).

Recombinant integrin αᵥβ₃ were coated on ELISA plates. Recombinant PKM2 along with test antibody or IgG (as control) were added into the integrin αᵥβ₃ coated plates. Plates were washed several times. Binding of the PKM2 to integrin αᵥβ₃ was measured using antibody against PKM2 (Fig. 14).

*Cell attachment assay.* Cell culture plates were coated with recombinant PKM2. HUVEC or COS-7 cells along with test antibody of IgG (as a control) ~ 2 - 5 µM were added to the plates. After 50 minutes incubation. The cells attached to the plates were counted (Fig. 18).

### Example 3: Extracellular PKM2 facilitates liver fibrosis progression

FIG 16A and FIG. 16B show that PKM2 facilitates liver fibrosis progression and the treatments with antibodies are capable of reducing the fibrosis. FIG. 16A illustrates a schematic schedule of TAA/alcohol liver fibrosis induction and subsequent rPKM2/rPKM1 treatments. FIG. 16A and 16B show that liver fibrosis is induced by TAA/alcohol and the animals were subsequently treated with indicated agents as shown in FIG. 16A. FIG. 16B shows Body weight changes during treatment course, (C) Liver weight of the animals at end point, (D) Representative liver images with enlarged call-out to show surface features. (E) Representative images of Sirius red stains of liver tissues from mice treated with indicated agents. The call-out is enlarged images showing the Sirius red stain feature. (F) Quantitation of Sirius red stains of sections of liver tissues from mice treated with indicated agents. Quantification was calculated from measurements of 10 mice. Four randomly selected tissue sections per animal and three randomly selected view fields in each section were quantified. The quantity of Sirius red stain is presented as % of total areas.

Liver fibrosis was induced with Balb/C mouse by i.p. administration of TAA and 10% ethanol in drinking water. Recombinant bacterially expressed PKM2 (rPKM2), PKM1 (rPKM1), or buffer was added into the fibrosis induction 15 days after first dose of TAA administration (FIG. 16A). At the end of experiments, animals were sacrificed. Animal body weights, liver weights, and out-surface of livers were closely examined. Clearly, addition of rPKM2 to the TAA-alcohol fibrosis induction significantly reduced mice body weights and increased liver sizes and weights (FIG. 16A and FIG. 16B ). Fibrotic features on the liver surface of animal group with rPKM2 addition were more apparent compared to those of without rPKM2 addition and those with addition of rPKM1. Sirius red stain of liver tissue sections demonstrated more and thicker collagen accumulation in the livers of animal group that was treated via rPKM2 addition. Dense collagen bends and cross-networks in livers of animals that were treated via rPKM2 addition indicated stronger liver cirrhosis.

### Example 4 - Antibody against PKM2 reverse liver fibrosis

A pair of rabbit monoclonal PKM2 antibodies was employed to disrupt the the PKM2-integrin interaction (16-2) - one was active and the other was not. The other one (21-3) is not (Figure 17A). Liver fibrosis of Balb/C mice was induced by treatment with TAA-alcohol. At end of fibrosis induction, purified antibodies from both hybridoma lines were administered at 4 mg/kg i.p. for 10 doses. At the end of treatment, animals were sacrificed. Animal body weights, liver weights, and out-surface of livers were examined. Antibody 16-2 treatment led to increase in mouse body weights and decrease in liver size and weight compared to those of IgG and 21-3 treated groups (Figure. 17B). Sirius red stain of liver tissue sections revealed substantial decrease in collagen contents and density, indicating resolve of collagen accumulation in fibrotic liver by the PKM2 antibody treatment.

Figure 17 shows PKM2 antibody reversed liver fibrosis (A) Attachment of LX-2 cells to rPKM2 coated plate in the presence of buffer, PKM2 antibody clone 16 (IgGPK16), PKM2 antibody clone 21 (IgGPK21), and LM609 (avb3Ab). The attachment is presented as total number of cells attached to the plate per view field. Control is the plate without rPKM2 coat. FIG. 17BLiver fibrosis is induced by TAA/alcohol and the animals were subsequently treated with indicated agents. FIG. 17BBody weight changes during treatment course, (C) Liver weight of the animals at end point, (D) Representative images of Sirius red stains of liver tissues from mice treated with indicated agents. The call-out is enlarged images showing the Sirius red stain feature.

### Example 5 Antibody against PKM2 reverse bleomycin induce lung fibrosis

Lung fibrosis of Balb/C mice was induced by Bleomycin treatment. At end of fibrosis induction (5 weeks), purified PKM2 antibody from hybridoma line were administered at 4 mg/kg i.p. for 10 doses. At the end of treatment, animals were sacrificed. Lungs from treated animals were collected and pictured (not shown). It is clear that bleomycin induced strong lung fibrosis. Fibrosis features on the lung surface of PKM2 antibody treated group were clearly less compared to that of IgG,buffer, and pirfenidone treated groups. Sirius red stain of lung tissue sections revealed substantial decrease (percent of total area) in collagen contents and density, indicating resolve of collagen accumulation in fibrotic lung by the PKM2 antibody treatment (Figure 18).

### Figure 18 PKM2 antibody reversed bleomycin induced lung fibrosis

Lung fibrosis is induced by bleomycin and the animals were subsequently treated with indicated agents. Quantitation of Sirius red stains of sections of lung tissues from mice treated with indicated agents. Quantification was calculated from measurements of 10 mice. Four randomly selected tissue sections per animal and three randomly selected view fields in each section were quantified. The quantity of Sirius red stain is presented as % of total areas. Normal is the samples from mice that were not induced fibrosis and also without subsequent treatment. Before treatment is the samples from mice that were sacrificed right after fibrosis induction without subsequent treatment. IgGPK is antibody against PKM2. IgG is purified rabbit IgG. Pirfenidone is a drug that was approved for lung fibrosis treatment by FDA.

Unless defined otherwise, all technical and scientific terms used herein have the same meanings as commonly understood by one of skill in the art to which the presently disclosed subject matter belongs. As used in this specification and the appended claims, the singular forms "a," "an" and "the" include plural referents unless the context clearly dictates otherwise.

## Claims

1. A composition comprising a pyruvate kinase isoform M2 (PKM2) antagonist for use in a method of treating a disease associated with aberrant integrin αvβ3 expression in a subject, wherein the method comprises administering to the subject an effective amount of the composition comprising a pyruvate kinase isoform M2 (PKM2) antagonist, **characterized in that** the PKM2 antagonist is an antibody inhibiting the binding of PKM2 to integrin αvβ3 wherein the disease is liver fibrosis or pulmonary fibrosis.

2. The composition for use according to claim 1, wherein the PKM2 antagonist is a monoclonal antibody that selectively binds PKM2.

3. The composition for use according to claim 1, wherein the monoclonal antibody comprises a heavy chain variable region and a light chain variable region, and wherein said antibody specifically binds to at least one PKM2 domain selected from a group consisting of domain A, domain B and domain C.

4. The composition for use according to claim 1, wherein the antibody is a neutralizing antibody or a humanized neutralizing antibody.

5. The composition for use according to claim 1, wherein the neutralizing antibody is a monoclonal antibody.

6. The composition for use according to claim 1, wherein the monoclonal antibody comprises a heavy chain variable region and a light chain variable region, and wherein said antibody specifically binds to at least one PKM2 domain selected from a group consisting of domain A, domain B and domain C.

## Patentansprüche

1. Zusammensetzung, die einen Pyruvatkinase-isoformen M2 (PKM2)-Antagonisten enthält, zur Verwendung in einem Verfahren zur Behandlung einer Krankheit, die mit einer aberranten Integrin avβ3-Expression in einem Subjekt verbunden ist, wobei das Verfahren das Verabreichen einer wirksamen Menge der Zusammensetzung, die einen Pyruvatkinase-isoformen M2 (PKM2)-Antagonisten beinhaltet, an das Subjekt umfasst, **dadurch gekennzeichnet, dass** der PKM2-Antagonist ein Antikörper ist, der die Bindung von PKM2 an Integrin αvβ3 hemmt, wobei die Krankheit Leberfibrose oder Lungenfibrose ist.

2. Zusammensetzung zur Verwendung gemäß Anspruch 1, wobei der PKM2-Antagonist ein monoklonaler Antikörper ist, der PKM2 selektiv bindet.

3. Zusammensetzung zur Verwendung gemäß Anspruch 1, wobei der monoklonale Antikörper eine variable Region der schweren Kette und eine variable Region der leichten Kette umfasst, und wobei der Antikörper spezifisch an mindestens eine PKM2-Domäne bindet, die aus einer Gruppe ausgewählt ist, die aus Domäne A, Domäne B und Domäne C besteht.

4. Die Zusammensetzung zur Verwendung gemäß Anspruch 1, wobei der Antikörper ein neutralisierender Antikörper oder ein humanisierter neutralisierender Antikörper ist.

5. Die Zusammensetzung zur Verwendung gemäß Anspruch 1, wobei der neutralisierende Antikörper ein monoklonaler Antikörper ist.

6. Zusammensetzung zur Verwendung gemäß Anspruch 1, wobei der monoklonale Antikörper eine variable Region der schweren Kette und eine variable Region der leichten Kette umfasst, und wobei der Antikörper spezifisch an mindestens eine PKM2-Domäne bindet, die aus einer Gruppe ausgewählt ist, die aus Domäne A, Domäne B und Domäne C besteht.

## Revendications

1. Composition comprenant un antagoniste de l'isoforme M2 de la pyruvate kinase (PKM2) à utiliser dans une méthode de traitement d'une maladie associée à une expression aberrante de l'intégrine αvβ3 chez un sujet, dans laquelle la méthode comprend l'administration au sujet d'une quantité efficace de la composition comprenant un antagoniste de l'isoforme M2 de la pyruvate kinase (PKM2), **caractérisée en ce que** l'antagoniste PKM2 est un anticorps inhibant la liaison de PKM2 à l'intégrine αvβ3 dans laquelle la maladie est une fibrose hépatique ou une fibrose pulmonaire.

2. La composition à utiliser selon la revendication 1, dans laquelle l'antagoniste de PKM2 est un anticorps monoclonal qui se lie sélectivement à PKM2.

3. La composition à utiliser selon la revendication 1, dans laquelle l'anticorps monoclonal comprend une région variable de chaîne lourde et une région variable de chaîne légère, et dans laquelle ledit anticorps se lie spécifiquement à au moins un domaine de PKM2 choisi dans un groupe constitué du domaine A, du domaine B et du domaine C.

4. La composition à utiliser selon la revendication 1, dans laquelle l'anticorps est un anticorps neutralisant ou un anticorps neutralisant humanisé.

5. La composition à utiliser selon la revendication 1, dans laquelle l'anticorps neutralisant est un anticorps monoclonal.

6. La composition à utiliser selon la revendication 1, dans laquelle l'anticorps monoclonal comprend une région variable de chaîne lourde et une région variable de chaîne légère, et dans laquelle ledit anticorps se lie spécifiquement à au moins un domaine PKM2 choisi dans un groupe constitué du domaine A, du domaine B et du domaine C.
